# EUROPEAN PATENT SPECIFICATION

(11) **EP 1 799 147 B1**
(45) Date of publication and mention of the grant of the patent: **15.02.2012**
(21) Application number: 05798028.6
(22) Date of filing: 20.09.2005
(51) Int. Cl.: A61F 2/01

(54) **ANTI-THROMBUS FILTER HAVING ENHANCED IDENTIFYING FEATURES**
ANTITHROMBUS-FILTER MIT VERBESSERTEN IDENTIFIKATIONSMERKMALEN
FILTRE ANTITHROMBOTIQUE A POSSIBILITES D'IDENTIFICATION AMELIOREES

(30) Priority: 20.09.2004 US 611415 P
(43) Date of publication of application: 27.06.2007
(73) Proprietor: Cook Medical Technologies LLC, Bloomington, IN 47404 (US)
(72) Inventor: SCHAEFFER, Darin, G., Bloomington, IN 47403 (US); PAUL, Ram, H., Jr., Bloomington, IN 47403 (US)
(74) Representative: Garratt, Peter Douglas
(86) International application number: PCT/US2005/033559
(87) International publication number: WO 2006/034233

(56) References cited:
- WO-A-01/15629
- US-A1- 2002 193 828
- US-B1- 6 267 776
- US-B1- 6 436 120

## Description

### CROSS-REFERENCE TO RELATED APPLICATIONS

This application claims the benefit of U.S. Provisional Application serial no. 60/611 ,415, filed on September 20, 2004, entitled "ANTI-THROMBOGENIC FILTER HAVING ENHANCED IDENTIFYING FEATURES,".

### BACKGROUND OF THE INVENTION

The present invention relates to medical devices. More particularly, the present invention relates to a removable filter having enhanced identifying features.

Filtering devices that are percutaneously placed in a body vessel, e.g., the vena cava, have been available for several years. A need for filtering devices arises in trauma patients, orthopedic surgery patients, neurosurgery patients, or in patients having medical conditions requiring bed rest or non-movement. During such medical conditions, the need for filtering devices arises due to the likelihood of thrombosis in the peripheral vasculature of patients wherein thrombi break away from the vessel wall, risking downstream embolism or embolization. For example, depending on the size, thrombi pose a serious risk of pulmonary embolism wherein blood clots migrate from the peripheral vasculature through the heart and into the lungs.

A filtering device may be deployed in a body vessel of a patient when, for example, anticoagulant therapy is contraindicated or has failed. Typically, filtering devices are permanent implants, each of which remains implanted in the patient for life, even though the condition or medical problem that required the device has passed. In more recent years, filters have been used or considered in preoperative patients and in patients predisposed to thrombosis which places the patient at risk for pulmonary embolism.

The benefits of filtering devices have been well established, but improvements may be made. For example, physicians have been challenged in monitoring the placement of filtering devices within the vasculature of a patient and monitoring the retrieval thereof. Although many filtering devices are comprised of material such as metal, physicians continue to experience difficulty in identifying filtering devices via fluoroscopy. It is useful for the physician to be able to determine the attitude and arrangement of a device when deployed within a body vessel of a patient such that adjustments may be made where needed. Thus, further mechanisms and features for identifying the device during deployment and retrieval without compromising the effectiveness of the filter are desired.

### BRIEF SUMMARY OF THE INVENTION

The present invention is set forth in the appended claims and generally provides a removable filter for capturing thrombi in a body vessel and having enhanced identifying and reduced endotheliosis features. One embodiment comprises a removable filter for capturing thrombi in a body vessel and having enhanced identifying and reduced endothelium features. The filter comprises an anti-blood clot portion disposed thereon to break thrombi and reduce the sizes thereof in the body vessel, an echogenic portion formed thereon for ultrasound detection during ultrasonography, and a radiopaque portion disposed thereon for enhanced placement and removal of the filter in the body vessel.

The removable filter comprises a plurality of struts having ends attached together. The struts have an anti-blood clot portion disposed thereon for reduced clots in the body vessel. The filter further includes a hub configured to axially house the first ends. The hub comprises a wall disposed about the struts to attach the first ends together. The wall includes an outer surface having an echogenic portion thereon for ultrasound detection. The filter further comprises a retrievable hook having a free end housed in the hub and extends oppositely from the struts to an arcuate portion having a radiopaque portion thereon for enhanced placement and removal of the filter in the blood vessel.

Further aspects, features, and advantages of the invention will become apparent from consideration of the following description and the appended claims when taken in connection with the accompanying drawings.

6436120 discloses a vena cava filter and placement set including features which can be visualized utilizing sonography. A method of implanting a vena cava filter employs sonography to enable the surgeon to direct the filter to a desired location and ensure that the filter is properly deployed. Designs include members of wire and slotted tubes, constructed of temperature-sensitive shape memory materials and of mechanically expanded materials.

US 2002193828 discloses an endovascular filter including a plurality of struts with distal ends adapted to anchor the filter to the vessel wall after deployment, such as by having barbs, the filter being adapted to be retrieved if desired. Strut distal ends are coated with an antiproliferative agent that inhibits the ingrowth of tissue around the filter, thereby permitting the filter to be retrieved and removed atraumatically after a prolonged period of time, thus extending the useful life of the retrievable filter. Optionally, the proximal end of the filter may also be so coated, or the entire filter.

### BRIEF DESCRIPTION OF THE DRAWINGS

Figure 1 is an environmental view of the filter in the vasculature of a patient in accordance with one embodiment of the present invention;

Figure 2a is a side perspective view of the filter in Figure 1;

Figure 2b is a cross-sectional view of the filter in Figure 2a taken along line 2b-2b;

Figure 3 is a side cross-sectional view of a blood vessel wherein the filter of Figure 2a is deployed;

Figure 4 is a cross-sectional view of the blood vessel of Figure 3 taken along line 4-4;

Figure 5a is a cross-sectional view of a blood vessel in which a retrieval sheath engages struts of the filter;

Figure 5b is a cross-sectional view of a blood vessel in which the retrieval sheath includes the filter in the collapsed state;

Figure 6 is a side perspective view of a filter in accordance with other embodiments of the present invention;

Figure 7a is a cross-sectional view of a strut of the filter taken along line 7-7 of Figure 6 in accordance with one embodiment of the present invention;

Figure 7b is a cross-sectional view of a strut of the filter taken along line 7-7 of Figure 6 in accordance with another embodiment of the present invention;

Figure 7c is a cross-sectional view of a strut of the filter taken along line 7-7 of Figure 6 in accordance with yet another embodiment of the present invention; and

Figure 7d is a cross-sectional view of a strut of the filter taken along line 7-7 of Figure 6 in accordance with still another embodiment of the present invention.

### DETAILED DESCRIPTION OF THE INVENTION

Embodiments of the present invention provide a filter for capturing thrombi and having anti-thrombogenic/fibrinolytic, echogenic and radiopaque features. The features of the filter provide for enhanced identifying and reduced endotheliosis in a body vessel of a patient. Generally, the anti-thrombogenic/fibrinolytic feature is preferably an anti-thrombogenic agent or a fibrinolytic agent disposed on the filter to decrease the rate of fibrin accumulation thereon and in the body vessel. For example, as clots are captured, the fibrinolytic agent breaks down the clots, lessening the time in which filter is needed in a body vessel. The echogenic feature preferably is comprised of marks formed on the filter that give rise to reflections of ultrasound waves during ultrasonography. The radiopaque feature is preferably a thin polymeric coating, ceramic coating, or noble metal coating applied on the filter for enhanced fluoroscopy.

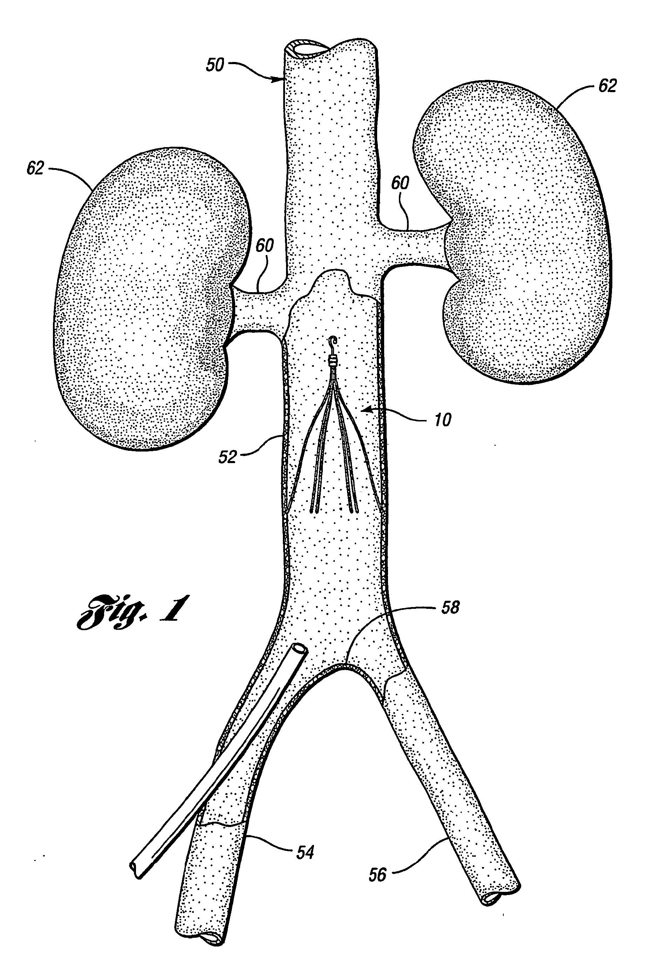

In accordance with one embodiment of the present invention, Figure 1 illustrates a filter 10. In this embodiment, filter 10 is a vena cava filter implanted in the vena cava 50 for the purpose of lysing or capturing thrombi carried by the blood flowing through the iliac veins 54, 56 toward the heart and into the pulmonary arteries. However, it is understood that filter may be any other filtering device implantable within any other vessel of a patient for distal protection without falling beyond the scope or spirit of the present invention. As shown, the iliac veins 54, 56 merge at juncture 58 into the vena cava 50. The renal veins 60 from the kidneys 62 join the vena cava 50 downstream of juncture 58. The portion of the vena cava 50, between the juncture 58 and the renal veins 60, defines the inferior vena cava 52 in which the filter 10 has been percutaneously deployed through one of the femoral veins. In this embodiment, the filter 10 preferably has a length smaller than the length of the inferior vena cava 52. In this example, if the lower part of the filter extends into the iliac veins, filtering effectiveness will be compromised and if the filter wires cross over the origin of the renal veins the filter wires might interfere with the flow of blood from the kidneys.

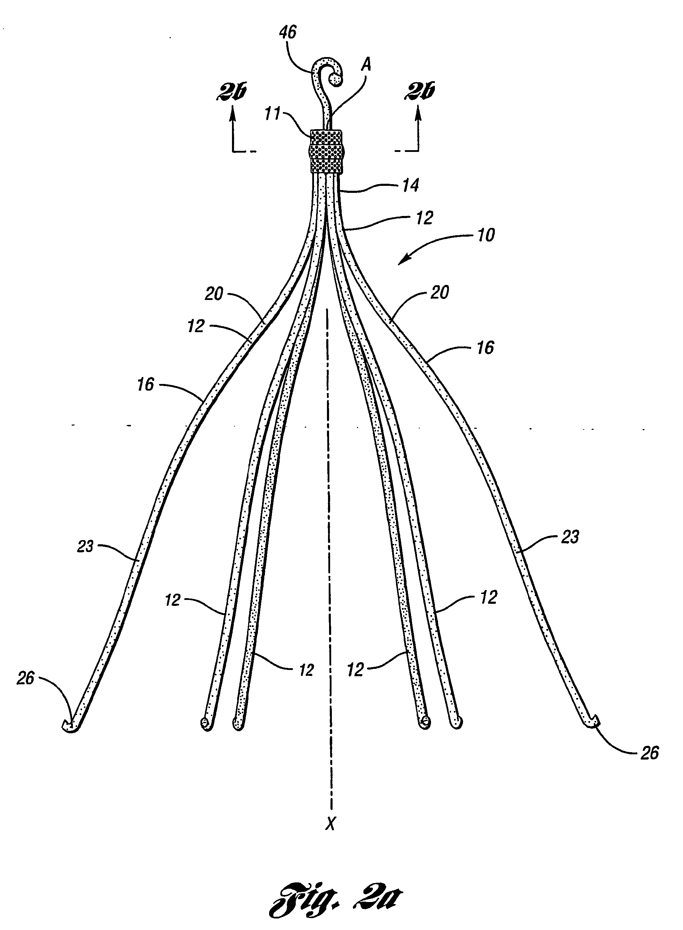

Figure 2a illustrates filter 10 in an expanded state and comprising six struts 12 each having first ends 14 that emanate from a hub 11. Hub 11 attaches by crimping first ends 14 of struts 12 together along a center point A in a compact bundle along a central or longitudinal axis X of the filter. In this embodiment, the struts 12 are preferably formed from wire having a round or near round cross-section with a diameter of at least about 0.015 inches. Of course, it is not necessary that the struts have a round cross-section. For example, the struts 12 could take on any shape with rounded edges to maintain non-turbulent blood flow. The length of the filter 10 is preferably defined by the length of a strut 12. Moreover, the diameter of the hub 11 is defined by the size of a bundle containing the struts 12.

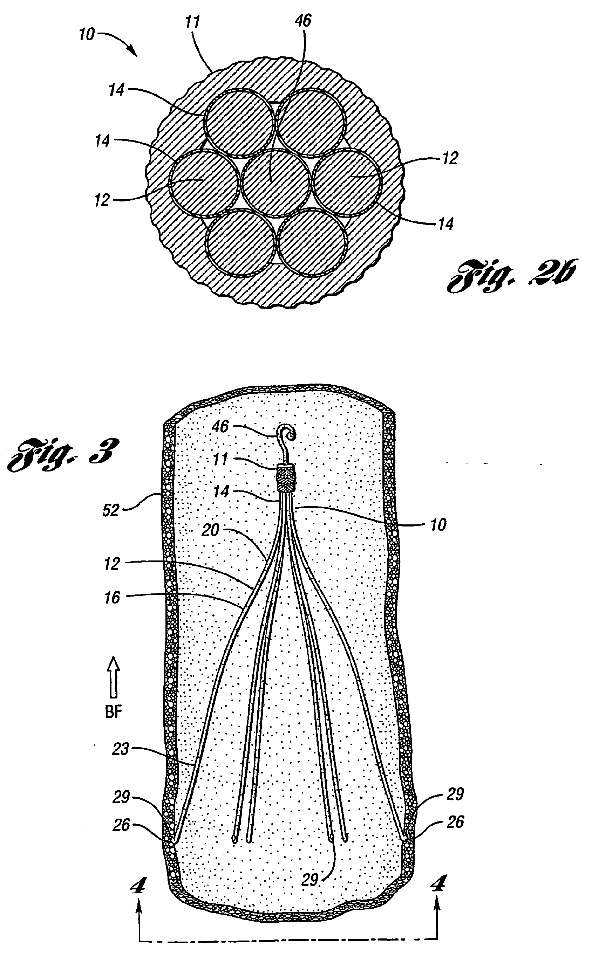

Figure 2b illustrates a cross-sectional view of the filter 10 of Figure 2a at hub 11. As shown in Figures 2a and 2b, the hub 11 houses a bundle of first ends 14 of the six struts 14. Figure 2b further depicts the configurations of the struts 12. In this embodiment, the struts 12 are spaced relatively evenly from on another with a retrieval hook 46 disposed at the center. As shown in Figures 2a and 2b, a retrieval hook 46 extends from the hub 11 opposite the plurality of struts 12 for removal of the filter 10 from a body vessel. Of course, any other configuration of the struts may be used without falling beyond the scope of the present invention as claimed.

As shown in Figure 2a, the struts 12 terminate at anchoring hooks 26 that will anchor in the vessel wall when the filter 10 is deployed at a delivery location in the vessel. The struts 12 are configured to move between an expanded state for engaging the anchoring hooks 26 with the vessel and a collapsed state for filter retrieval or delivery.

In this embodiment, each of the struts 12 includes a coating, at least one of an anti-thrombogenic coating and a fibrinolytic coating, disposed thereon for reduced blood clots and endotheliosis in the body vessel. The coating is preferably an anti-thrombogenic agent that acts to inhibit formation of blood clots or a fibrolytic agent to dissolve fibrin by enzymatic action. The coating may include heparin, streptokinase, urokinase, alteplase, anistreplase, prourokinase, reteplase, tenecteplase, lanoteplase, staphylokinase, alfimeprase, lumbrokinase, nattokinase, boluoke, serrapeptase, and euglobulin or any other suitable anti-thrombogenic agent or fibrinolytic agent. For example, as known, heparin is a medication typically used to reduce the likelihood of blood clots from forming in a patient's body.

In use, thrombi are caught by the configuration (mentioned below) formed by the struts. As thrombi are lodged within the struts, the coating assists in reducing the formation of blood clots or in breaking down blood clots or thrombi on contact. The coating may be applied onto the struts by any suitable means, such as by spraying or dipping. The coating may then be cured for a predetermined time known in the art.

As shown, hub 11 includes echogenic marks or dimples formed thereon to provide reflections of ultrasound waves during ultrasonograpy, *e.g*. two-dimensional or three-dimensional ultrasonography. Preferably, the echogenic marks are formed circumferentially about hub 11. After deployment of the filter in a body vessel of a patient, the filter may be monitored using ultrasonography. The hub 11 may be identified by way of the echogenic marks thereon and may further assist in determining the location of the retrieval hook 46 during retrieval or delivery of the filter.

As shown in Figures 2a and 3, filter 10 further includes a radiopaque coating disposed on the retrieval hook 46. The radiopaque coating may be a polymeric coating, ceramic coating, or noble metal coating applied on the retrieval hook 46 for enhanced fluoroscopy. In this embodiment, the radiopaque coating comprises a noble metal coating. Noble metals that may be used as the radiopaque coating include gold, platinum, iridium, palladium, or rhodium, or a mixture thereof. The radiopaque coating may be applied to the retrieval hook by any suitable means, e.g., spraying or dipping. The radiopaque feature of the filter provides enhanced fluoroscopy to more easily identify the retrieval hook during delivery, adjustment, or retrieval of the filter from the vasculature of the patient.

Each strut 12 includes an arcuate segment 16 having a soft S-shape. Each arcuate segment 16 is formed with a first curved portion 20 that is configured to softly bend away from the longitudinal or central axis X of the filter 10 and a second curved portion 23 that is configured to softly bend toward the longitudinal axis of the filter 10. Due to the soft bends of each arcuate segment 16, a prominence or a point of inflection on the strut 12 is substantially avoided to aid in non-traumatically engaging the vessel wall.

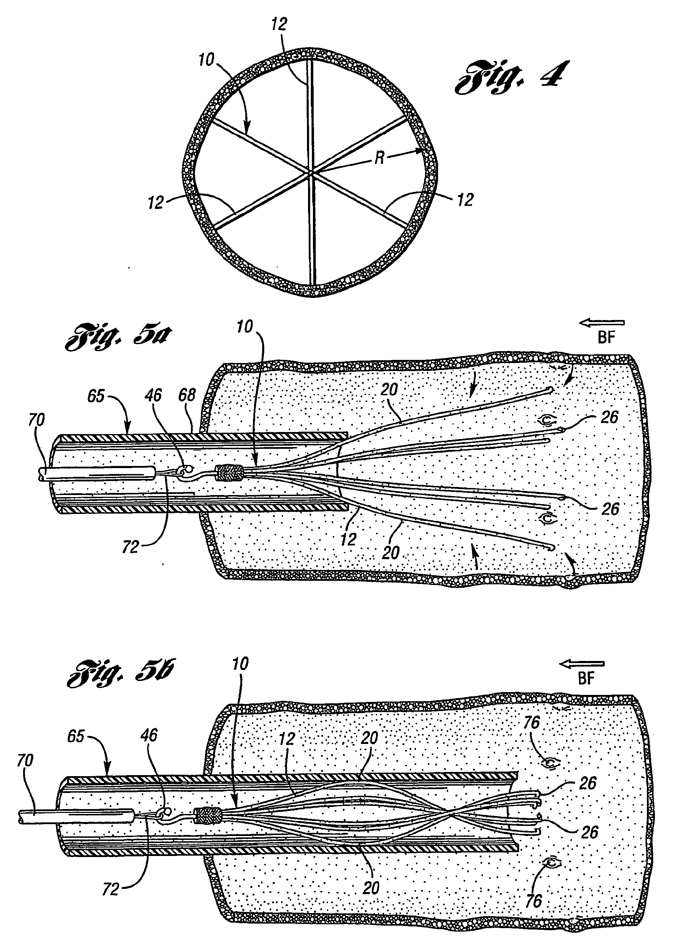

In the expanded state, each arcuate segment 16 extends arcuately along a longitudinal axis (as shown in Figure 2a) and linearly relative to a radial axis R (as shown in Figure 4) from the first end 14 to the anchoring hook 26. The struts 12 extend linearly relative to the radial axis and avoid entanglement with other struts. In this embodiment, the filter 10 extends longitudinally as shown in Figure 2a, defining the longitudinal axis X of filter 10. The filter 10 further radially expands and collapses as shown in Figure 4, defining the radial axis R of the filter 10.

When the filter 10 is deployed in a blood vessel, the anchoring hooks 26 engage the walls of the blood vessel to define a first axial portion to secure the filter in the blood vessel. The anchoring hooks 26 prevent the filter 10 from migrating from the delivery location in the blood vessel where it has been deposited. The struts 12 are shaped and dimensioned such that, when the filter 10 is freely expanded, the filter 10 may have a diameter of between about 25 mm and 45 mm and a length of between about 3 cm and 7 cm in this embodiment. For example, the filter 10 may have a diameter of about 35 mm and a length of about 5 cm. The struts 12 have sufficient spring strength that when the filter is deployed the anchoring hooks 26 will anchor into the vessel wall.

Figure 5b illustrates the filter 10 in a collapsed state disposed in a delivery/retrieval tube 65 for delivery or retrieval. As shown, the filter 10 is shaped for each strut 12 to cross another strut 12 relative to the longitudinal axis X. As a result, in the collapsed state, the anchoring hooks 26 are configured to invert or inwardly face the longitudinal axis X for retrieval and delivery of the filter 10. This inverted or inwardly facing configuration of the anchoring hooks 26 allows for simplified delivery and retrieval of filter 10.

In the collapsed state, each strut 12 is configured to cross another strut 12 relative to the longitudinal axis X such that the arcuate segments 16, first curved portions 20 or second curved portions 23, occupy a first diameter. In this embodiment, the first diameter is greater than a second diameter occupied by the anchoring hooks 26 for filter retrieval or delivery. It has been found that the first diameter of the arcuate segments 16 serves to clear a path of retrieval, reducing radial force from the sheath or blood vessel on the anchoring hooks 26 during removal of the filter 10 from a patient. Reducing the radial force on the anchoring hooks 26 assists in preventing the anchoring hooks 26 from scraping, scratching, or tearing the inner wall of a sheath during removal of the filter 10 from a patient.

It is to be noted that the filter 10 may be delivered or retrieved by any suitable introducer (delivery or retrieval) tube. For example, the introducer tube may have an inside diameter of between about 4.5 French and 16 French, and more preferably between about 6.5 French and 14 French.

Figure 3 illustrates the filter 10 expanded after being deployed in inferior vena cava 52 in this embodiment. As shown, the inferior vena cava 52 has been broken away so that the filter 10 can be seen. The direction of the blood flow BF is indicated in Figure 3 by the arrow that is referred to as BF. The anchoring hooks 26 at the ends of the struts 12 are shown as being anchored in the inner lining of the inferior vena cava 52. The anchoring hooks 26 include barbs 29 that, in one embodiment, project toward the hub 11 of the filter. The barbs 29 function to retain the filter 10 in the location of deployment. In Figure 3, the filter 10 is inserted through the proximal end of the delivery tube with the removal hook 46 leading and anchoring hooks 26 of the struts 12 held by a filter retainer member for delivery via the femoral vein of a patient.

In one embodiment, the filter 10 may be inserted through the proximal end of the delivery tube with the anchoring hooks of the struts leading and the removal hook trailing for delivery via the jugular vein of a patient. In this embodiment, a pusher wire having a pusher member at its distal end may be fed through the proximal end of the delivery tube thereby pushing the filter until the filter reaches the distal end of the delivery tube to a desired location.

When the filter 10 is fully expanded in the body vessel, the anchoring hooks 26 of the struts 12 are in engagement with the vessel wall. The anchoring hooks 26 of the struts 12 have anchored the filter 10 at the location of deployment in the vessel, preventing the filter 10 from moving with the blood flow through the vessel. As a result, the filter 10 is supported by two sets of struts that are spaced axially along the length of the filter.

Figure 3 illustrates the filter 10 fully expanded after being deployed in inferior vena cava 52. As shown, the inferior vena cava 52 has been broken away so that the filter 10 can be seen. The direction of the blood flow BF is indicated in Figure 3 by the arrow that is labeled BF. The anchoring hooks 26 at the ends of the struts 12 are shown as being anchored in the inner lining of the inferior vena cava 52. The anchoring hooks 26 include barbs 29 that, in one embodiment, project toward the hub 11 of the filter. The barbs 29 function to retain the filter 10 in the location of deployment. The spring biased configuration of the struts 12 further causes the anchoring hooks 26 to engage the vessel wall and anchor the filter at the location of deployment. After initial deployment, the pressure of the blood flow on the filter 10 contributes in maintaining the barbs 29 anchored in the inner lining of the inferior vena cava 52.

As seen in Figure 3, the hub 11 and retrieval hook 46 are positioned downstream from the location at which the anchoring hooks 26 are anchored in the vessel. In one embodiment wherein the anti-blood clot portion comprises an anti-thrombogenic agent, the anti-blood clot portion inhibits or reduces the formation of thrombus and endotheliosis on the filter. In another embodiment wherein the anti-blood clot portion comprises a fibrinolytic agent, the anti-blood clot portion breaks down fibrin and thrombus when captured by the struts. Further, remaining thrombi may remain lodged in the filter for removal. The filter 10 along with thrombi captured therein may then be percutaneously removed from the vessel. When the filter 10 is to be removed, the retrieval hook 46 is preferably grasped by a retrieval instrument that is percutaneously introduced in the body vessel in the direction of retrieval hook 46 first.

Figure 4 depicts a netting configuration or pattern formed by the struts 12 and the hub 11 relative to radial axis R. The netting pattern shown in Figure 4 functions to catch thrombi carried in the blood stream prior to reaching the heart and lungs to prevent the possibility of a pulmonary embolism. The netting pattern is sized to catch and stop thrombi that are of a size that are undesirable to be carried in the vasculature of the patient. Due to its compacted size, the hub minimally resists blood flow.

Figure 4 depicts the netting pattern including struts at substantially equal angular space relative to each other. The netting pattern provides an even distribution between the struts to the blood flow, increasing the likelihood of capturing thrombi. However, it is to be understood that each of the sets of struts may be configured in any other suitable manner relative to radial axis R.

Figures 5a and 5b illustrates part of a retrieval device 65 being used in a procedure for removing the filter 10 from the inferior vena cava 52. In this example, the retrieval device 65 is percutaneously introduced into the superior vena cava via the jugular vein. In this procedure, a removal catheter or sheath 68 of the retrieval device 65 is inserted into the superior vena cava. A wire 70 having a loop snare 72 at its distal end is threaded through the removal sheath 68 and is exited through the distal end of the sheath 68. The wire 70 is then manipulated by any suitable means from the proximal end of the retrieval device such that the loop snare 72 captures the removal hook 46 of the filter 10. Using counter traction by pulling the wire 70 while pushing the sheath 68, the sheath 68 is passed over the filter 10. As the sheath 68 passes over the filter 10, the struts 12 engage the edge of the sheath 68 and are caused to pivot or undergo bend deflection at the hub 11 toward the longitudinal axis of the filter. The pivoting toward the longitudinal axis causes the ends of the struts 12 and 30 to be retracted from the vessel wall. In this way, small point lesions 76 on the vessel wall are created in the removal procedure. As shown, the small point legions 76 are created by the anchoring hooks 26 of the struts 12. However, it is to be noted that any other suitable procedure may be implemented to remove the filter from the patient.

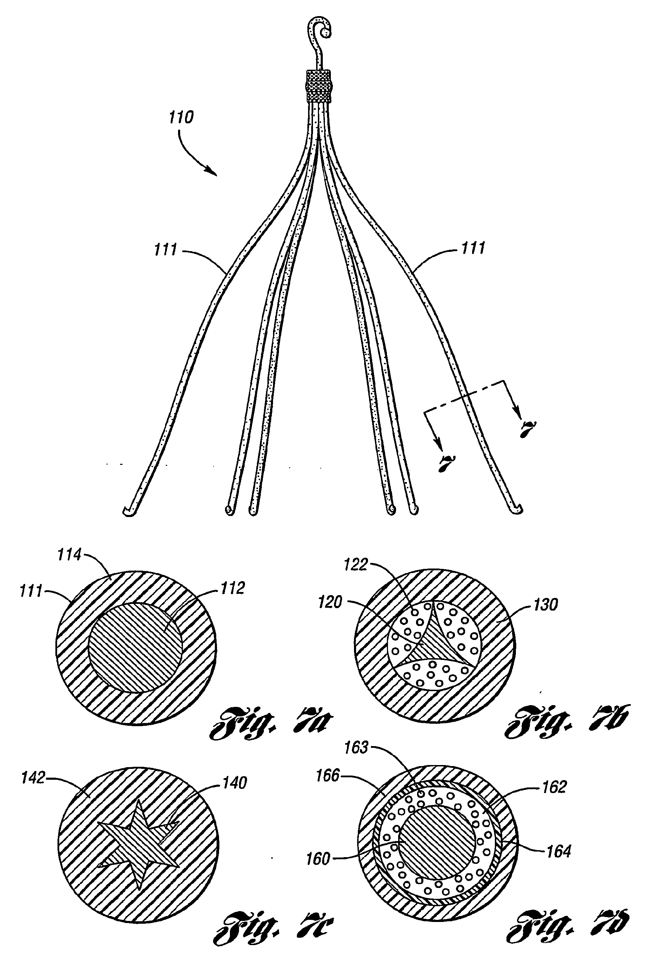

Figure 6 illustrates a filter 110 in accordance with other embodiments of the present invention. Figures 7a-7d are cross-sectional views of different profiles of strut 111 depicted in Figure 4. In this embodiment, the profile of the strut may have a plurality of layers providing enhanced identifying features. In one embodiment, Figure 7a illustrates the strut having a noble metal core 112 for radiopacity and a rigid polymeric outer layer 114 disposed about the noble metal core 112. Noble metals that may be used include gold, platinum, iridium, palladium, or rhodium, or a mixture thereof. The outer layer 114 may be made of any suitable rigid polymeric material.

In another embodiment, the struts of the filter may include a reflective core to provide reflections of ultrasound waves during ultrasonograpy. Figures 7b and 7c depict struts having reflective cores. In one embodiment, Figure 7b illustrates a reflective core 120 about which a first polymeric layer 122 is disposed. The reflective core 120 may be made of reflective material suitable for ultrasonography such as metal or metal alloy. In this example, the first polymeric 122 layer includes hollow cavities 124 formed therein for enhanced ultrasonography. The first polymeric layer 122 may include polyethylene, polypropylene, or any other suitable polymeric material. A second polymeric layer 130 is disposed about the first polymeric layer 122. The second polymeric layer 130 may include polyethylene, polypropylene, or any other suitable polymeric material.

Figure 7c illustrates another embodiment of the cross-sectional profile of the strut. In Figure 7c, the strut includes a reflective core 140 having a profile different than the cross-sectional profile depicted in Figure 7b. The reflective core 140 may be made of reflective material suitable for ultrasonography such as metal or metal alloy. The reflective core 140 has an outer layer 142 disposed thereabout. The outer layer 142 may include the same material as the second polymeric layer 130 of Figure 7b.

In another embodiment depicted in Figure 7d, the profile of the struts may include a metallic or rigid polymeric core 160 coated with an ecogenic layer 162 having hollow ecogenic particles 163 for enhanced ultrasonography. In this embodiment, the metallic or rigid core 160 may be made of the same material as the outer layer 114 of Figure 7a. The ecogenic layer 162 may include any suitable polymeric material. In this embodiment, a drug eluting layer 164 is disposed about the ecogenic layer 162 for drug eluting capabilities. In this embodiment, any suitable eluting drug or system for eluting drugs may be used, e.g., paclitaxel, docetaxel, sirolimus, everolimus, or other immunosuppressants. The drug eluting layer 164 is coated with a permeable polymeric layer 166 to allow the eluting drug to be dispersed therethrough for the desired treatment.

The filter 10 may be comprised of any suitable material such as a superelastic material, stainless steel wire, cobalt-chromium-nickel-molybdenum-iron alloy, or cobalt-chrome alloy. It is understood that the filter 10 may be formed of any other suitable material that will result in a self-opening or self-expanding filter, such as shape memory alloys. Shape memory alloys have a property of becoming rigid, that is, returning to a remembered state, when heated above a transition temperature. A shape memory alloy suitable for the present invention may comprise Ni-Ti available under the more commonly known name Nitinol. When this material is heated above the transition temperature, the material undergoes a phase transformation from martensite to austenic, such that material returns to its remembered state. The transition temperature is dependent on the relative proportions of the alloying elements Ni and Ti and the optional inclusion of alloying additives.

In one alternate embodiment, the filter 10 may be made from Nitinol with a transition temperature that is slightly below normal body temperature of humans, which is about 98.6°F. Although not necessarily a preferred embodiment, when the filter 10 is deployed in a body vessel and exposed to normal body temperature, the alloy of the filter 10 will transform to austenite, that is, the remembered state, which for one embodiment of the present invention is the expanded configuration when the filter 10 is deployed in the body vessel. To remove the filter 10, the filter 10 is cooled to transform the material to martensite which is more ductile than austenite, making the filter 10 more malleable. As such, the filter 10 can be more easily collapsed and pulled into a lumen of a catheter for removal.

In another alternate embodiment, the filter 10 may be made from Nitinol with a transition temperature that is above normal body temperature of humans, which is about 98.6° F. Although not necessarily a preferred embodiment, when the filter 10 is deployed in a body vessel and exposed to normal body temperature, the filter 10 is in the martensitic state so that the filter 10 is sufficiently ductile to bend or form into a desired shape, which for the present invention is an expanded configuration. To remove the filter 10, the filter 10 is heated to transform the alloy to austenite so that the filter 10 becomes rigid and returns to a remembered state, which for the filter 10 in a collapsed configuration.

While the present invention has been described in terms of preferred embodiments, it will be understood, of course, that the invention is not limited thereto since modifications may be made to those skilled in the art, particularly in light of the foregoing teachings.

## Claims

1. A removable filter for capturing thrombi in a body vessel and having enhanced identifying and reduced endothelium features, the filter comprising one of a fibrinolytic coating and an anti-thrombogenic coating for reduced thrombi in the body vessel, an echogenic portion formed thereon for ultrasound detection during ultrasonography, and a radiopaque portion disposed thereon for enhanced placement and removal of the filter in the body vessel, further comprising a plurality of struts having first ends attached together, each strut having an arcuate segment extending from the first end to an anchoring hook, the struts being configured to move between an expanded state for engaging the anchoring hooks with the body vessel and a collapsed state for filter retrieval or delivery, said coating being disposed on the struts; a hub configured to axially house the first ends, the hub comprising a wall disposed about the struts to attach the first ends together, the walls including the outer surface having the echogenic portion disposed thereon; and a retrieval hook having a free end housed in the hub, the retrieval hook extending oppositely from the struts to an arcuate portion and having the radiopaque portion disposed thereon and wherein each of the struts comprises a first layer being a metallic reflective core for enhanced identification of the filter, each of the struts further comprising a second layer disposed about the first layer, the second layer comprising a polymeric material.

2. The removable filter of claim 1 wherein said coating comprises: heparin, streptokinase, urokinase, alteplase, anistreplase, prourokinase, reteplase, tenecteplase, lanoteplase, staphylokinase, alfimeprase, or euglobulin or a mixture thereof.

3. The removable filter of claim 1 wherein the arcuate segment includes a first curved portion and a second curved portion, the first curved portion extending from the first end, the second curved portion extending from the first curved portion and terminating at the anchoring hook.

4. The removable filter of claim 3 wherein the first curved portion is configured to extend radially from the central axis of the filter and the second curved portion is configured to extend radially toward the central axis of the filter.

5. The removable filter of claim 1 wherein each strut is formed of at least one of the following materials: a superelastic material, stainless steel wire, Nitinol, elgiloy, chronichrome, or cobalt chrome alloy.

6. The removable filter of claim 1 wherein the struts are configured to pivot at the first ends thereof to move between the collapsed and expanded states.

7. The removable filter of claim 1 wherein the echogenic portion includes a plurality of marks formed about the hub for providing reflections of ultrasound waves during ultrasonography.

8. The removable filter of claim 1 wherein the radiopaque portion is a radiopaque coaling applied to the retrieval hook for enhanced fluoroscopy.

9. The removable filter claim 8 wherein the radiopaque coating includes a polymeric coating, a ceramic coating, and a noble metal coating.

10. The removable filter of claim 9 wherein the noble metal coating includes gold, platinum, iridium, palladium, or rhodium, or a mixture thereof.

11. The removable filter of claim 1, wherein each of the struts further comprising a drug eluting layer disposed about the core, the drug eluting layer comprising polymeric material and an eluting drug, each strut comprising an outer layer through which the eluting drug is dispersed for treatment to the body vessel.

## Patentansprüche

1. Entfernbarer Filter zum Fangen von Thromben in einem Körpergefäß mit verbesserten Identifikations- und verringerten Endothel-Merkmalen, wobei der Filter eine fibrinolytische Beschichtung oder eine antithrombogene Beschichtung zur Reduzierung von Thromben im Körpergefäß, einen darauf geformten echogenen Abschnitt zum Ultraschallnachweis während einer Ultraschalluntersuchung und einen darauf angeordneten röntgenopaken Abschnitt zur leichteren Platzierung und Entfernung des Filters im Körpergefäß umfasst, und wobei er ferner eine Vielzahl von Streben mit aneinander befestigten ersten Enden umfasst, wobei jede Strebe ein bogenförmiges Segment aufweist, das sich vom ersten Ende zu einem Verankerungshaken erstreckt, wobei sich die Streben zwischen einem expandierten Zustand zum Eingriff der Verankerungshaken in das Körpergefäß und einem kollabierten Zustand zur Rückholung oder Ablage des Filters bewegen können, wobei die Beschichtung auf den Streben angeordnet ist; einen Ansatz zur axialen Unterbringung der ersten Enden, wobei der Ansatz eine um den Streben angeordnete Wand umfasst, um die ersten Enden aneinander zu befestigen, wobei die Wände die Außenseite mit dem darauf angeordneten echogenen Abschnitt aufweisen; und einen Rückholhaken mit einem im Ansatz untergebrachten freien Ende umfasst, wobei sich der Rückholhaken von der gegenüberliegenden Seite der Streben bis zu einem bogenförmigen Abschnitt mit dem darauf angeordneten röntgenopaken Abschnitt erstreckt und worin jede der Streben eine erste Schicht umfasst, die ein metallischer Reflexionskern zur verbesserten Identifikation des Filters darstellt, wobei jede der Streben ferner eine auf der ersten Schicht angeordnete zweite Schicht umfasst, wobei die zweite Schicht ein Polymermaterial umfasst.

2. Entfernbarer Filter nach Anspruch 1, worin die Beschichtung Folgendes umfasst: Heparin, Streptokinase, Urokinase, Alteplase, Anistreptase, Prourokinase, Reteplase, Tenecteplase, Lanoteplase, Staphylokinase, Alfimeprase oder Euglobin oder ein Gemisch davon.

3. Entfernbarer Filter nach Anspruch 1, worin das bogenförmige Segment einen ersten gebogenen Abschnitt und einen zweiten bogenförmigen Abschnitt aufweist, wobei sich der erste gebogene Abschnitt vom ersten Ende erstreckt und sich der zweite gebogene Abschnitt sich vom ersten gebogenen Abschnitt erstreckt und am Verankerungshaken endet.

4. Entfernbarer Filter nach Anspruch 3, worin sich der erste gebogene Abschnitt radial von der Mittelachse des Filters erstrecken kann und sich der zweite gebogene Abschnitt radial zur Mittelachse des Filters erstrecken kann.

5. Entfernbarer Filter nach Anspruch 1, worin jede Strebe zumindest aus einem der folgenden Materialien geformt ist: ein superelastisches Material, Edelstahldraht, Nitinol, Elgiloy, Chronichrom oder Kobalt-Chrom-Legierung.

6. Entfernbarer Filter nach Anspruch 1, worin die Streben an ihren ersten Enden schwenkbar sind, um sich zwischen dem kollabierten Zustand und dem expandierten Zustand zu bewegen.

7. Entfernbarer Filter nach Anspruch 1, worin der echogene Abschnitt eine Vielzahl von Markierungen aufweist, die um den Ansatz geformt sind, um Reflexionen von Ultraschallwellen während einer Ultraschalluntersuchung zu liefern.

8. Entfernbarer Filter nach Anspruch 1, worin der röntgenopake Abschnitt eine röntgenopake Beschichtung ist, die zur besseren fluoroskopischen Sicht auf dem Rückholhaken aufgebracht ist.

9. Entfernbarer Filter nach Anspruch 8, worin die röntgenopake Beschichtung eine Polymerbeschichtung, eine Keramikbeschichtung und eine Edelmetallbeschichtung aufweist.

10. Entfernbarer Filter nach Anspruch 9, worin die Edelmetallbeschichtung Gold, Platin, Iridium, Palladium oder Rhodium oder ein Gemisch davon aufweist.

11. Entfernbarer Filter nach Anspruch 1, worin jede der Streben ferner eine um den Kern angeordnete Arzneimittel freisetzende Schicht umfasst, wobei die Arzneimittel freisetzende Schicht ein Polymermaterial und eine freizusetzendes Arzneimittel umfasst, wobei jede Strebe eine Außenschicht umfasst, durch die das freizusetzende Arzneimittel zur Behandlung des Körpergefäßes dispergiert.

## Revendications

1. Filtre amovible pour capturer des thrombus dans un vaisseau corporel et ayant des fonctionnalités d'identification et d'endothélium réduit améliorées, le filtre comprenant l'un d'un revêtement fibrinolytique et d'un revêtement antithrombogène pour réduire les thrombus dans le vaisseau corporel, une partie échogène formée sur celui-ci pour la détection échographique pendant une échographie, et une partie radio-opaque disposée sur celui-ci pour un placement et un retrait améliorés du filtre dans le vaisseau corporel, comprenant en outre une pluralité de supports ayant des premières extrémités attachées conjointement, chaque support ayant un segment arqué s'étendant de la première extrémité à un crochet d'ancrage, les supports étant configurés pour se déplacer entre un état déployé pour engager les crochets d'ancrage avec le vaisseau corporel et un état rétracté pour le retrait ou le placement du filtre, ledit revêtement étant disposé sur les supports ; un moyeu configuré pour loger axialement les premières extrémités, le moyeu comprenant une paroi disposée autour des supports pour raccorder les premières extrémités conjointement, les parois comprenant la surface extérieure ayant la partie échogène disposée sur celle-ci ; et un crochet d'extraction ayant une extrémité libre logée dans le moyeu, le crochet d'extraction s'étendant dans la direction opposée depuis les supports vers une partie arquée et ayant la partie radio-opaque disposée sur celui-ci et où chacun des supports comprend une première couche étant un noyau réfléchissant métallique pour l'identification améliorée du filtre, chacun des supports comprenant en outre une deuxième couche disposée autour de la première couche, la deuxième couche comprenant un matériau polymère.

2. Filtre amovible de la revendication 1 dans lequel ledit revêtement comprend : l'héparine, la streptokinase, l'urokinase, l'altéplase, l'anistréplase, la pro-urokinase, le rétéplase, la ténectéplase, la lanotéplase, la staphylokinase, l'alfiméprase, ou l'euglobuline ou un mélange de celles-ci.

3. Filtre amovible de la revendication 1 dans lequel le segment arqué comprend une première partie courbée et une deuxième partie courbée, la première partie courbée s'étendant depuis la première extrémité, la deuxième partie courbée s'étendant depuis la première partie courbée et terminant au niveau du crochet d'ancrage.

4. Filtre amovible de la revendication 3 dans lequel la première partie courbée est configurée pour s'étendre radialement depuis l'axe central du filtre et la deuxième partie courbée est configurée pour s'étendre radialement vers l'axe central du filtre.

5. Filtre amovible de la revendication 1 dans lequel chaque support est formé d'au moins l'un des matériaux suivants : un matériau superélastique, un câble d'acier inoxydable, Nitinol, Eigiloy, Chronichrome, ou un alliage cobalt-chrome.

6. Filtre amovible de la revendication 1 dans lequel les supports sont configurés pour pivoter aux premières extrémités de ceux-ci pour se déplacer entre les états rétracté et déployé.

7. Filtre amovible de la revendication 1 dans lequel la partie échogène comprend une pluralité de marques formées autour du moyeu pour produire des réflexions d'ondes ultrasonores pendant une échographie.

8. Filtre amovible de la revendication 1 dans lequel la partie radio-opaque est un revêtement radio-opaque appliqué sur le crochet d'extraction pour fluoroscopie améliorée.

9. Filtre amovible de la revendication 8 dans lequel le revêtement radio-opaque comprend un revêtement polymère, un revêtement de céramique, et un revêtement de métal noble.

10. Filtre amovible de la revendication 9 dans lequel le revêtement de métal noble comprend de l'or, du platine, de l'iridium, du palladium, ou du rhodium, ou un mélange de ceux-ci.

11. Filtre amovible de la revendication 1, dans lequel chacun des supports comprend en outre une couche d'élution de médicament disposée autour du noyau, la couche d'élution de médicament comprenant un matériau polymère et un médicament d'élution, chaque support comprenant une couche externe dans laquelle le médicament d'élution est dispersé pour traitement du vaisseau corporel.
